# EUROPEAN PATENT SPECIFICATION

(11) **EP 4 199 712 B1**
(45) Date of publication and mention of the grant of the patent: **25.06.2025**
(21) Application number: 21763071.4
(22) Date of filing: 18.08.2021
(51) Int. Cl.: A01M 1/20

(54) **DEVICE FOR EVAPORATING VOLATILE SUBSTANCES**
VORRICHTUNG ZUR VERDAMPFUNG VON FLÜCHTIGEN SUBSTANZEN
DISPOSITIF D'ÉVAPORATION DE SUBSTANCES VOLATILES

(30) Priority: 21.08.2020 EP 20192097
(43) Date of publication of application: 28.06.2023
(73) Proprietor: Zobele Holding SpA, 38100 Trento (IT)
(72) Inventor: DEFLORIAN, Stefano, 38100 TRENTO (IT); SORDO, Walter, 38100 TRENTO (IT)
(74) Representative: Herrero & Asociados, S.L.
(86) International application number: PCT/EP2021/072930
(87) International publication number: WO 2022/038182

(56) References cited:
- EP-A1- 1 709 980
- WO-A1-2018/121857
- GB-A- 2 516 925
- JP-A- S59 106 249
- JP-U- S5 572 878

## Description

The present invention refers to a device for evaporating volatile substances, comprising a porous mat impregnated with volatile substances and a heating element that heats the porous mat for evaporating said volatile substances.

### Background of the invention

Electrical volatile substance diffusers have become a common consumer product in the last decades. Most common devices are connected to the electric mains and comprise a refill containing a liquid with volatile substances and a wick that transports the liquid from the inside of the refill to the external part of the wick, where the volatile particles evaporate, or a porous mat impregnated with the volatile substances. This refill or porous mat is placed in a device that with the help of an electrical heater promotes the evaporation of the volatile substance.

Standard technology for the heater construction deal about either an electrical heater embedded in a ceramic casing or a nude electrical resistor placed inside a plastic housing. The ceramic casing and the plastic housing have the function to assure electrical insulation between the resistor and the surroundings.

The existing solutions for electrical devices for evaporating volatile substances have several drawbacks.

Firstly, they have generally big dimensions and each of their surfaces dissipate heat. Therefore, only part of the heat is directed towards the wick or porous mat where it is needed. That leads to a lower electrical efficiency of the device, that makes it especially inadequate to satisfy one of the recurrent consumer requests that is portability. Portability is of special interest for insecticides substance diffuser, in order to give protection against bugs bites in outdoor conditions.

Secondly, the heat not directed towards the wick or porous mat promotes a general heating of the device that can rise substantially the temperature of the surface of the device. This is the reason why the devices use to have a considerable dimension in order to avoid the temperature at the surface exceed a limit defined by electrical certification entities. Here, again, bigger dimension of the device is a limitation for portability.

JPS5572878U discloses a transpirator comprising a planar heater with perforated holes vertically penetrated beneath a drug impregnated mat.

JPS59106249A discloses a high-frequency transmitting heat insulating material provided with a partially cut-out portion or a partially thin-walled portion serving as a heat-dissipating passage for fumigation heating of the fumigated insecticidal substrate impregnated with the insecticidal component absorbs the high frequency and easily absorbs the high frequency.

WO2018121857A1 discloses a device for evaporating volatile substances comprises a wick impregnated with volatile substances and a heating element that heats the wick for evaporating said volatile substances.

### Description of the invention

Therefore, the objective of the present invention is to provide a device for evaporating volatile substances that permit to direct substantially all the heat provided by the heating element to the porous mat, preventing an excessive rising of the temperature of the surface of the device.

With the device of the invention said drawbacks can be solved, presenting other advantages that will be described hereinafter.

The device for evaporating volatile substances according to the present invention is defined in claim 1, and it comprises:
- a porous mat impregnated with volatile substances,
- a heating element that heats the porous mat for evaporating the volatile substances, and a reflective element for reflecting the heat from the heating element to said porous mat,
wherein the reflective element forms a cavity defined by a bottom and side walls where the porous mat and the heating element are placed.

According to a preferred embodiment, the reflective element is formed from a metal sheet, e.g. from a mirror sheet.

Advantageously, the cavity formed by the reflective element comprises one or more seals for making the cavity airtight.

Still furtherly advantageously, the cavity airtight is having a certain level of vacuum, preferably from 0.1-0.2 bar.

According to a preferred embodiment, the heating element is a ceramic heater.

Advantageously, the porous mat is placed on the heating element, and the device also comprises a printed circuit board where the reflective element is mounted, and the printed circuit board is preferably fed by a battery.

Still advantageously, there is an air gap between the heater and the porous mat, to allow the evaporation from this side of the porous mat, that requires less energy, preferably from 0.1 to 3 mm, more preferably between 1 and 2 mm.

Preferably, there is a gap between the printed circuit board and the reflective element.

Furthermore, the device for evaporating volatile substances according to the present invention also comprises a grill that covers the porous mat, through which the volatile substances can evaporate and that also serves as a protection against accidental contact with the porous mat, which is hot during its use.

Advantageously, the porous mat can comprise a holder for facilitating its handling.

Thanks to this feature, the reflective element reflects or redirects the heat radiation from the heating element back towards the porous mat where the volatile substances are impregnated, and the heat energy that is going away from the porous mat is reflected back to the porous mat, so that a heating element which consumes less energy can be used for the same evaporation capacity.

Furthermore, as the cavity formed by the reflective element is airtight, a more efficient heating of the porous mat is achieved. This is especially of interest for portable devices that can be exposed to uncontrolled situations where dirt may be introduced accidentally inside the device. Any matter present in the cavity will affect heat transfer, due on one side to its thermal inertia, and on the other side, due to its interaction to heat transfer irradiation between heater and reflective material.

### Brief description of the drawings

For a better comprehension of what has been disclosed, some drawings are attached in which, diagrammatically and only as a non-limitative example, a practical embodiment is shown.
Fig. 1 is an exploded perspective view of the components forming the device for evaporating volatile substances according to the present invention; and
Figs. 2 and 3 are perspective views during the placement of the porous mat of the device for evaporating volatile substances according to the present invention.

### Description of a preferred embodiment

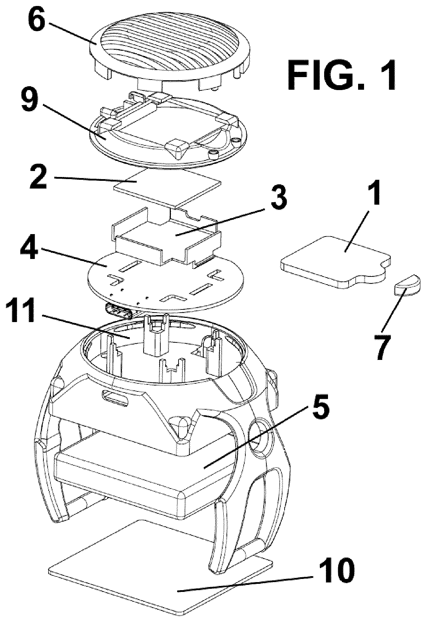

The device of Fig. 1. is an example of portable device, with the highest level of requirement for a portable device, as it is a band wrist mounted device. The requirement, as anticipated earlier are: to be lightweight, to be adequate to be use in areas where dirt or water can be present, and to have low level of external temperature of the housing.

The device for evaporating volatile substances according to the present invention comprises a porous mat 1 which is impregnated with volatile substances, such as fragrances or insecticides, and a heating element 2 that heats the porous mat 1 for evaporating the volatile substances.

The heating element 2 is any suitable heating element, but according to a preferred embodiment it is a ceramic heater, having a flat shape as shown in Fig. 1.

The porous mat 1 has also a flat shape and preferably comprises a holder 7 that facilitates its handing, because the porous mat 1 is used as a refill, so that it can be replaced once all the volatile substances have been evaporated.

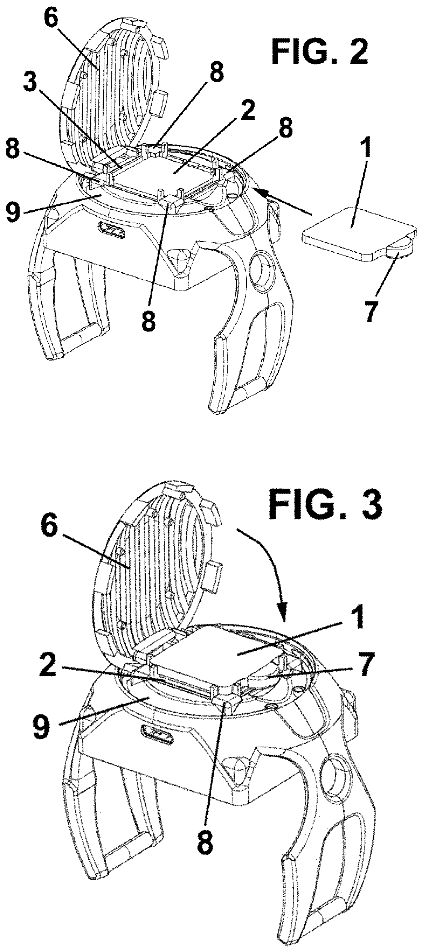

In the use position, shown in Fig. 3, the porous mat 1 is placed on the heating element 2 with a distance between them.

The device according to the present invention also comprises a reflective element 3 for reflecting the heat from the heating element 2 to the porous mat 1, and the reflective element 3 forms a cavity defined by a bottom and side walls where the porous mat 1 and the heating element 2 are placed.

According to the shown embodiment, the reflective element 3 is formed from a metal sheet, e.g. a mirror sheet, and the cavity formed by the reflective element 3 comprises one or more seals 8 for making the cavity airtight. In the shown embodiment, the seals 8 are four, each placed at the corners of the cavity, which has a rectangular plan.

The device for evaporating volatile substances according to the present invention also comprises a printed circuit board 4 where the reflective element 3 is mounted, leaving a gap between the printed circuit board 4 and the reflective element 3.

The printed circuit board 4 is fed by a battery 5, that can be connected to the mains for recharging it.

The printed circuit board 4 heats the heating element 2 placed inside the cavity defined by the reflective element 3, and this heating element 2 heats the porous mat 1 for the evaporation of the volatile substances.

The assembly of the printed circuit board 4, the reflective element 3 and the heating element 2 are mounted inside a housing 11, which can have a watch-like shape, comprising a closure 10 for accessing to the battery 5.

Furthermore, the housing 11 also comprises a grill 6 which is hinged to the rest of the housing 11 for permitting the insertion and the removal of the porous mat 1. This grill 6 also has the function of protecting the users from contacting accidentally the heated elements.

According to the shown embodiment, the grill 6 is mounted on an intermediate housing 9 that surrounds the reflective element 3.

The operation of the device according to the present invention is very simple. Firstly, a porous mat 1 must be placed in the use position, as shown in Figs. 2 and 3, and the device must be switched on.

Then the battery will feed the printed circuit board 4, which will heat the heating element 2. This heating element 2 will heat directly and indirectly the porous mat 1, and all the heat from the heating element 2 will be concentrated and reflected to the porous mat 1 by the reflective element 3.

Even though reference is made to a specific embodiment of the invention, it is clear for a person skilled in the art that the disclosed device is susceptible of variations and modifications without departing from the scope of protection defined by the attached claims.

## Claims

1. Device for evaporating volatile substances, comprising:
- a porous mat (1) impregnated with volatile substances,
- a heating element (2) that heats the porous mat (1) for evaporating the volatile substances, and
a reflective element (3) for reflecting the heat from the heating element (2) to said porous mat (1),
the reflective element (3) forming a cavity defined by a bottom and side walls where the porous mat (1) and the heating element (2) are placed,
**characterized in that** the device also comprises a printed circuit board (4) that heats the heating element (2) placed inside the cavity defined by the reflective element (3).

2. Device for evaporating volatile substances according to claim 1, wherein the reflective element (3) is formed from a metal sheet.

3. Device for evaporating volatile substances according to claim 1 or 2, wherein the reflective element (3) is formed from a mirror sheet.

4. Device for evaporating volatile substances according to anyone of the previous claims, wherein the cavity formed by the reflective element (3) comprises one or more seals for making the cavity airtight.

5. Device for evaporating volatile substances according to claim 4, wherein the cavity formed by the reflective element (3) has an internal vacuum in the range of -0.1 to -0.2 bar.

6. Device for evaporating volatile substances according to claim 1, wherein the heating element (2) is a ceramic heater.

7. Device for evaporating volatile substances according to claim 1, wherein the porous mat (1) is placed on the heating element (2).

8. Device for evaporating volatile substances according to claim 1, wherein the porous mat (1) is placed at a distance of 1 to 2 mm from the heating element (2).

9. Device for evaporating volatile substances according to claim 7, wherein there is a gap between the printed circuit board (4) and the reflective element (3).

10. Device for evaporating volatile substances according to claim 7 or 8, wherein the printed circuit board (4) is fed by a battery (5).

11. Device for evaporating volatile substances according to anyone of the previous claims, also comprising a grill (6) that covers the porous mat (1).

12. Device for evaporating volatile substances according to claim 1, wherein the porous mat (1) comprises a holder (7).

## Patentansprüche

1. Vorrichtung zum Verdampfen flüchtiger Substanzen, umfassend:
- eine poröse Matte (1), die mit flüchtigen Substanzen imprägniert ist,
- ein Heizelement (2), das die poröse Matte (1) zum Verdampfen der flüchtigen Substanzen erwärmt,
und
ein reflektierendes Element (3) zum Reflektieren der Wärme vom Heizelement (2) zu der porösen Matte (1),
wobei das reflektierende Element (3) einen Hohlraum bildet, der durch einen Boden und Seitenwände begrenzt ist, in dem die poröse Matte (1) und das Heizelement (2) platziert sind,
**dadurch gekennzeichnet, dass** die Vorrichtung außerdem eine Leiterplatte (4) umfasst, die das Heizelement (2) erwärmt, das in dem durch das reflektierende Element (3) begrenzten Hohlraum platziert ist.

2. Vorrichtung zum Verdampfen flüchtiger Substanzen gemäß Anspruch 1, wobei das reflektierende Element (3) aus einem Metallblech gebildet ist.

3. Vorrichtung zum Verdampfen flüchtiger Substanzen gemäß Anspruch 1 oder 2, wobei das reflektierende Element (3) aus einer Spiegelplatte gebildet ist.

4. Vorrichtung zum Verdampfen flüchtiger Substanzen gemäß einem der vorstehenden Ansprüche,
wobei der durch das reflektierende Element (3) gebildete Hohlraum eine oder mehrere Dichtungen umfasst, um den Hohlraum luftdicht zu machen.

5. Vorrichtung zum Verdampfen flüchtiger Substanzen gemäß Anspruch 4, wobei der durch das reflektierende Element (3) gebildete Hohlraum ein Innenvakuum im Bereich von -0,1 bis -0,2 bar hat.

6. Vorrichtung zum Verdampfen flüchtiger Substanzen gemäß Anspruch 1, wobei das Heizelement (2) ein Keramik-Heizelement ist.

7. Vorrichtung zum Verdampfen flüchtiger Substanzen gemäß Anspruch 1, wobei die poröse Matte (1) auf dem Heizelement (2) platziert ist.

8. Vorrichtung zum Verdampfen flüchtiger Substanzen gemäß Anspruch 1, wobei die poröse Matte (1) in einem Abstand von 1 bis 2 mm vom Heizelement (2) platziert ist.

9. Vorrichtung zum Verdampfen flüchtiger Substanzen gemäß Anspruch 7, wobei zwischen der Leiterplatte (4) und dem reflektierenden Element (3) ein Spalt vorhanden ist.

10. Vorrichtung zum Verdampfen flüchtiger Substanzen gemäß Anspruch 7 oder 8, wobei die Leiterplatte (4) von einer Batterie (5) gespeist wird.

11. Vorrichtung zum Verdampfen flüchtiger Substanzen gemäß einem der vorhergehenden Ansprüche, die außerdem ein Gitter (6) umfasst, das die poröse Matte (1) abdeckt.

12. Vorrichtung zum Verdampfen flüchtiger Substanzen gemäß Anspruch 1, wobei die poröse Matte (1) einen Halter (7) umfasst.

## Revendications

1. - Dispositif d'évaporation de substances volatiles, comprenant :
- un tapis poreux (1) imprégné de substances volatiles,
- un élément chauffant (2) qui chauffe le tapis poreux (1) pour évaporer les substances volatiles, et
- un élément réflecteur (3) pour refléter la chaleur de l'élément chauffant (2) vers ledit tapis poreux (1),
l'élément réflecteur (3) formant une cavité définie par un fond et des parois latérales où le tapis poreux (1) et l'élément chauffant (2) sont placés,
**caractérisé en ce que** le dispositif comprend également une carte de circuit imprimé (4) qui chauffe l'élément chauffant (2) placé à l'intérieur de la cavité définie par l'élément réflecteur (3).

2. - Dispositif d'évaporation de substances volatiles selon la revendication 1, dans lequel l'élément réflecteur (3) est constitué d'une feuille de métal.

3. - Dispositif d'évaporation de substances volatiles selon la revendication 1 ou 2, dans lequel l'élément réflecteur (3) est constitué d'une feuille miroir.

4. - Dispositif d'évaporation de substances volatiles selon l'une quelconque des revendications précédentes, dans lequel la cavité formée par l'élément réflecteur (3) comprend un ou plusieurs joints pour rendre la cavité étanche.

5. - Dispositif d'évaporation de substances volatiles selon la revendication 4, dans lequel la cavité formée par l'élément réflecteur (3) a un vide interne compris entre -0,1 et -0,2 bar.

6. - Dispositif d'évaporation de substances volatiles selon la revendication 1, dans lequel l'élément chauffant (2) est un appareil de chauffage en céramique.

7. - Dispositif d'évaporation de substances volatiles selon la revendication 1, dans lequel le tapis poreux (1) est placé sur l'élément chauffant (2).

8. - Dispositif d'évaporation de substances volatiles selon la revendication 1, dans lequel le tapis poreux (1) est placé à une distance de 1 à 2 mm de l'élément chauffant (2).

9. - Dispositif d'évaporation de substances volatiles selon la revendication 7, dans lequel se trouve un espace entre la carte de circuit imprimé (4) et l'élément réflecteur (3).

10. - Dispositif d'évaporation de substances volatiles selon la revendication 7 ou 8, dans lequel la carte de circuit imprimé (4) est alimentée par batterie (5).

11. - Dispositif d'évaporation de substances volatiles selon l'une quelconque des revendications précédentes, comprenant également une grille (6) qui recouvre le tapis poreux (1).

12. - Dispositif d'évaporation de substances volatiles selon la revendication 1, dans lequel le tapis poreux (1) comprend un support (7).
